# EUROPEAN PATENT SPECIFICATION

(11) **EP 1 146 866 B1**
(45) Date of publication and mention of the grant of the patent: **29.11.2006**
(21) Application number: 00904556.8
(22) Date of filing: 25.01.2000
(51) Int. Cl.: A61K 31/19, A61P 17/10, A61K 31/21, A61K 31/045

(54) **A METHOD FOR TREATING PATIENTS WITH ACNE BY ADMINISTERING A CYCLIC GMP PDE INHIBITOR**
BEHANDLUNGSMETHODE VON AKNEPATIENTEN DURCH VERABREICHUNG VON HEMMERN DER CYCLISCHEN GMP PDE
TRAITEMENT DE L'ACNE A BASE D'INHIBITEUR DE PHOSPHODIESTERASE PDE DE GMP CYCLIQUE

(30) Priority: 29.01.1999 US 239840; 20.08.1999 US 378660
(43) Date of publication of application: 24.10.2001
(73) Proprietor: OSI Pharmaceuticals, Inc., Melville, NY 11747 (US)
(72) Inventor: MENANDER, Kerstin, B., Meadowbrook, PA 19046 (US); MAYLE, Mark, Jeffrey, San Antonio, TX 78240 (US)
(74) Representative: Baldock, Sharon Claire
(86) International application number: PCT/US2000/001840
(87) International publication number: WO 2000/044372

(56) References cited:
- WO-A-94/01407
- US-A- 3 532 752
- US-A- 5 011 843
- US-A- 5 401 774
- US-A- 5 500 230
- US-A- 5 643 959

## Description

This application claims priority under 35 U.S.C. §120 to United States Patent Application Serial No. 09/378,660 filed August 20, 1999, and to United States Patent Application Serial No. 09/239,840 filed January 29, 1999.

### BACKGROUND OF THE INVENTION

Acne is a skin disease that often scars those afflicted, and can afflict patients at young ages -- typically teen years -- when their self-images are the most sensitive. The scarring is commonly permanent even if the condition is treated with medications. Some patients experience symptoms well into their adult years.

Acne is believed to be caused typically when the sebaceous glands become clogged due to skin cell debris and an excess of a specific type of skin bacteria. Sebaceous glands are located within the dermis layer of the skin along the hair shaft. Keratin and other chemicals associated with the skin can clog the hair shaft and the sebaceous gland. The bacteria, *Propionibacterium acnes* (*P. acnes*), which is always present, multiplies to a much greater degree when the sebaceous glands are clogged because the bacteria prefer an anaerobic environment, which is present when the glands become clogged.

*P. acnes* produces a lipase enzyme that hydrolyzes triglycerides of the sebaceous gland into free fatty acids. The fatty acids along with bacterial proteins and keratin can irritate the skin tissues. This may lead to an inflammatory response and the formation of an acne lesion.

Although the exact cause of acne is unknown, hormones, genetics, and environmental factors all seem to play a role. Androgens, such as testosterone, play an important role in the development of acne lesions. This is evidenced by the correlation between the onset of puberty and the development of acne, and the fact that acne is generally more severe in males than in females.

The type of acne treatments currently recommended depend on the type and the severity of the acne. A few over-the-counter medications (e.g., benzoyl peroxide and salicylic acid) can be used for mild forms of acne, but are ineffective for the moderate and severe cases of acne.

Tretinoin (Retin-A) is often used in combination with or as a replacement for treatment with benzoyl peroxide. Tretinoin is a derivative of vitamin A and is available for topical use. It acts to prevent comedone formation through its anti-keratinization effect. The side effects of tretinoin include heightened sensitivity to exposure to sunlight and local irritation.

Topical antibiotics are sometimes used for treatment of patients with mild to moderate inflammatory acne, but are ineffective against more severe inflammatory acne. To the extent antibiotics have effects against more severe (i.e. inflammatory) forms of acne, the antibiotics must be systemically administered, usually at high doses (e.g., 500 to 2,000 mg/day) initially, followed by maintenance doses (e.g., 250 to 500 mg/day). The most commonly prescribed antibiotic is tetracycline, but erythromycin and minocycline are also used. Such antibiotics, particularly at high doses can lead to side effects including gastrointestinal symptoms such as nausea, vomiting, abdominal pain, diarrhea, rashes, and other allergic reactions. Tetracyclines also can cause fetal harm if used during pregnancy.

Hormone therapy is another approach to treatment of acne based on the involvement of androgens in the development of acne. Estrogen therapy can be effective, but its usefulness is particularly limited in males by side effects such as gynecomastia, suppression of the testes, and uncertain effects on skeletal growth.

Isotretinoin (ACCUTANE)™ has been used in the treatment of severe nodulocystic acne. ACCUTANE^{™} therapy can lead to improvement in acne and can, in some cases, exhibit the potential to prevent most permanent scarring from inflammatory acne. The severe side effects of ACCUTANE^{™}, however, are often prohibitive. Dry skin, dry eyes, headache, hair thinning, musculoskeltal pain, and other complications may result from treatment with ACCUTANE^{™}. Due to its teratogenicity, ACCUTANE^{™} should not be used by women who are pregnant or might become pregnant.

Thus, to date, there is typically a trade-off between efficacy and side effects, particularly in the treatment of more severe forms of acne. Accordingly, an effective treatment of acne, particularly its more severe forms, with clinically insignificant side effects is desired.

### BRIEF SUMMARY OF THE INVENTION

This invention relates to a method for treating acne by administering to an afflicted patient a therapeutically effective amount of a cyclic GMP (cGMP) phosphodiesterase (PDE) inhibitor, preferably an inhibitor of PDE5 and/or PDE2. With such an inhibitor, acne can be treated with minimal side effects associated with conventional acne medications.

### DETAILED DESCRIPTION OF THE INVENTION

The cGMP PDE inhibitor according to the present invention is a compound of Formula I: 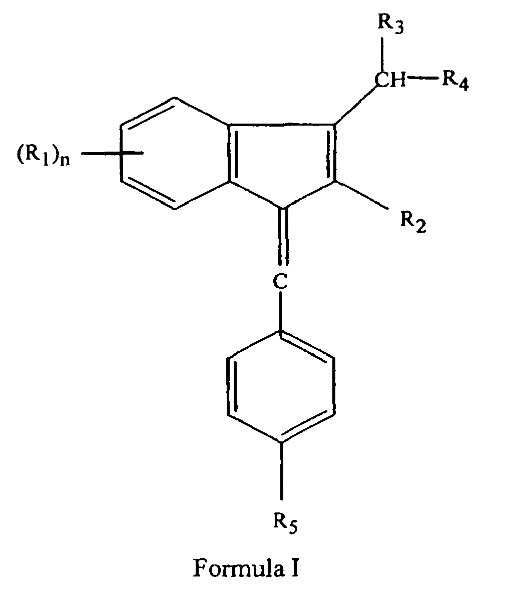 wherein R₁ is independently selected in each instance from the group consisting of hydrogen, halogen, alkoxy having 1 to 8 carbons, hydroxy, alkyl having 1 to 8 carbons, alkyl mercapto having 1 to 8 carbons, alkylsulfonyl having 1 to 8 carbons, alkylamino having 1 to 8 carbons, di-alkyl amino having 1 to 8 carbons, amino, nitro, nitrile, alkyl carboxylate having 1 to 8 carbons, -CO₂H, and sulfonamido;
R₂ is selected from the group consisting of hydrogen and alkyl having 1 to 8 carbons;
R₃ is selected from the group consisting of hydrogen, alkyl having 1 to 8 carbons, hydroxy, and amino;
R₄ is selected from the group consisting of -COM and CH₂OH wherein M is selected from the group consisting of hydroxy, substituted alkoxy having 1 to 8 carbons, amino, alkylamino, dialkylamino, N-morpholino, hydroxyalkylamino, polyhydroxyamino, dialkylaminoalkylamino, aminoalkyl amino, and the group OMe, wherein Me is a cation;
R₅ is an alkyl sulfonyl; and n is an integer from 0 to four;
or a pharmaceutically acceptable salt thereof.

Preferred compounds within the scope of Formula I include those wherein R₁ is halogen; n is 1; R₂ is alkyl having 1 to 8 carbons; M is hydroxy; and R₃ is hydrogen or alkyl having 1 to 8 carbons. More preferred compounds useful in the therapeutic method of this invention include those wherein R₁ is 5-fluoro; n is 1; R₂ is methyl; M is hydroxy; and R₃ is hydrogen.

The most preferred compound of Formula I for treating acne is exisulind. The chemical formula of exisulind is (Z)-5-fluoro-2-methyl-1-(p-methylsulfonyl-benzylidene)-3-indenyl acetic acid. The cGMP-specific PDE inhibitory characteristics of exisulind are reported in U.S. Patent No. 5,858,694. Clinical studies have demonstrated that exisulind is generally well tolerated by patients, has no reported clinically relevant side effects, and can be used safely by humans.

As used herein, the term "acne" includes the various known types of acne and related skin disorders, including acne vulgaris, acne conglobata, acne fulminans, pyoderma facaile, acne keloidalis, chloracne, and steroid acne. The most common form of acne is acne vulgaris, which is characterized by two types of lesions, inflammatory and noninflammatory. Noninflammatory lesions include open comedones (blackheads) and closed comedones (whiteheads) and are characterized by a lymphocytic infiltrate. Inflammatory lesions can be superficial or deep. Superficial inflammatory lesions include papules and pustules, and deep inflammatory lesions consist of cysts and nodules. Inflammatory lesions include those characterized by rupture of the follicular wall and aggregation of neutrophils and mononuclear cells. Acne vulgaris and the other forms of acne which may be treated by administering a therapeutically effective amount of a compound of Formula I are described in more detail in Principles and Practice of Dermatology, Chapter 70: Acne and Related Disorders, W. Mitchell Sams, Jr. and Peter J. Lynch eds., 1990.

As used herein, the term "alkyl" refers to straight, branched or cyclic alkyl groups and to substituted aryl alkyl groups.

The term "alkoxy having 1 to 8 carbons" refers to alkoxy groups having from 1 to 8 carbons, including straight, branched or cyclic arrangements.

The term "alkylmercapto having 1 to 8 carbons" refers to a sulfide group that is substituted with a alkyl group having 1 to 8 carbons, and the term "alkyl sulfonyl having 1 to 8 carbons" refers to a sulfone group that is substituted with a alkyl group having 1 to 8 carbons.

The term "alkyl carboxylate having 1 to 8 carbons" refers to a carboxylate group that is substituted with a alkyl group having 1 to 8 carbons.

The term "pharmaceutically acceptable salt" refers to non-toxic acid addition salts and alkaline earth metal salts of the compounds of Formula I. The salts can be prepared *in situ* during the final isolation and purification of such compounds, or separately by reacting the free base or acid functions with a suitable organic acid or base, for example. Representative acid addition salts include the hydrochloride, hydrobromide, sulfate, bisulfate, acetate, valerate, oleate, palmatate, stearate, laurate, borate, benzoate, lactate, phosphate, tosylate, mesylate, citrate, maleate, fumarate, succinate, tartrate, glucoheptonate, lactobionate, lauryl sulfate salts. Representative alkali and alkaline earth metal salts include the sodium, calcium, potassium and magnesium salts.

It will be appreciated that certain compounds of Formula I can possess an asymmetric carbon atom and are thus capable of existing as enantiomers. Unless otherwise specified, this invention includes such enantiomers, including any racemates. The separate enantiomers may be synthesized from chiral starting materials, or the racemates can be resolved by conventional procedures that are well known in the art of chemistry such as chiral chromatography, fractional crystallization of diastereomeric salts and the like.

Compounds of Formula I also can exist as geometrical isomers (Z and E); the Z isomer is preferred.

As noted above, preferred compounds within the scope of Formula I include those wherein R₁ is halogen; n is 1; R₂ is alkyl having 1 to 8 carbons, M is hydroxy; and R₃ is hydrogen or alkyl having 1 to 8 carbons. More preferred compounds useful in treating acne include those wherein R₁ is 5-fluoro; n is 1; R₂ is methyl; M is hydroxy; and R₃ is hydrogen.

The most preferred compound of Formula I for treating acne is exisulind, (Z)-5-fluoro-2-methyl-1-(p-methylsulfonylbenzylidene)-3-indenyl acetic acid or its pharmaceutically acceptable salts.

Cyclic GMP PDEs include the GMP-binding, cyclic GMP-specific phosphodiesterase (designated cGB-PDE) which is a phosphodiesterase gene family 5 isoenzyme (hereinafter "PDE5"). PDE5 is described more fully by Beavo, et al., in U.S. Patent Nos. 5,652,131 and 5,702, 936. Another cGMP PDE is the cGMP-stimulated PDE (designated cGS-PDE) which is a phosphodiesterase gene family 2 isoenzyme (hereinafter "PDE2"). PDE2 is described more fully by Martins, et al., in *J. Biol. Chem.,* 257: 1973-1979, 1982, and by Yang, et al., in *Biochem. Biophys. Res. Comm.,* 205: 1850-1858, 1994.

The term "cGMP PDE" as used herein refers to enzymes such as PDE2 and PDE5 and any of their isoforms.

The term "cGMP PDE inhibition" refers to inhibition of at least PDE2, or inhibition of at least PDE5, or inhibition of at least a combination of the two types of enzymes. The term "cGMP PDE inhibitor" refers to an inhibitor that inhibits the activity of at least PDE2, or that inhibits the activity of at least PDE5, or that inhibits the activity of at least a combination of the two types of enzymes.

In clinical studies for uses other than acne, it has been demonstrated that exisulind can be safely administered to human patients. In studies with adult patients, patients with intact colons received exisulind at a dosage of 400 mg/day, and patients who had previously undergone a colectomy received exisulind at a dosage of 600 mg/day. In another study with adult patients, the patients received 500 mg/day of exisulind. In a study involving pediatric patients, the dosage of exisulind used ranged from 250 to 350 mg per day.

In these studies, exisulind is generally well tolerated in patients. There have been no clinically relevant side effects reported. The maximum tolerated dose (MTD) was determined to be 600 mg/day in patients with colectomies, and 400 mg/day in the group with intact colons. The lower MTD in the patients with intact colons is due to some of the patients experiencing a moderate, reversible, asymptomatic elevation in liver function tests. These elevations were completely reversible on stopping the drug. Additionally, after a patient with elevated liver enzymes stops receiving the drug for approximately one week, the patient can then safely resume treatment at the same or a lower therapeutic dose, i.e. the dosages mentioned above. The elevations seem to be dose related and have been seen mostly at doses above the maximum tolerated dose. There is one group of 18 patients who received exisulind at a daily dose of 600 mg/day for an extended period of time with no such clinically significant side effects. Thirteen of those patients received exisulind for over three years.

Other examples of cGMP-specific PDE inhibitors include MY-5445 and dipyridamole.

Additional compounds that may be useful in the practice of this invention include those disclosed in GB 2 063 249 A, GB 2 063 249 A, EP 0 607 439 A1, US 4,101,548, US 4,001,238, US 4,001,237, US 3,920,636, US 4,060,615, WO 97/03985, EP 0 607 439 A1, US 4,101,548, US 4,001,238, US 4,001,237, US 3,920,636, US 4,060,615, WO 97/03985, EP 0 395 328, US 4,209,623, EP 0 395 328, US 4,209,623, US 5,354,571, EP 0 428 268 A2, US 5,354,571, EP 0 428 268 A2, 807,826, US 3,031,450, US 3,322,755, US 5,401,774, 807,826, US 3,031,450, US 3,322,755, US 5,401,774, US 5,147,875, PCT WO 93/12095, US 5,147,875, PCT WO 93/12095, US 4,885,301, WO 93/07149, EP 0 349 239 A2, EP 0 352 960 A2, EP 0 526 004 A1, EP 0 463 756 A1, US 4,885,301, WO 93/07149, EP 0 349 239 A2, EP 0 352 960 A2, EP 0 526 004 A1, EP 0 463 756 A1, EP 0 607 439 A1, EP 0 607 439 A1, WO 94/05661, EP 0 351 058, US 4,162,316, EP 0 347 146, US 4,047,404, US 5,614,530, US 5,488,055, WO 97/03985, , WO 97/03675, WO 95/19978, US 4,880,810, , WO 98/08848, US 5,439,895, US 5,614,627, PCT US94/01728, WO 98/16521, EP 0 722 943 A1, , EP 0 722 937 A1, EP 0 722 944 A1, WO 98/17668, WO 97/24334, WO 97/24334, WO 97/24334, WO 97/24334, WO 97/24334, WO 98/06722, PCT/JP97/03592, WO 98/23597, WO 94/29277, WO 98/14448, WO 97/03070, WO 98/38168, WO 96/32379, and PCT/GB98/03712.

Compounds useful in the practice of this invention may be formulated into pharmaceutical compositions together with pharmaceutically acceptable carriers for oral administration in solid or liquid form, or for intravenous, intramuscular, subcutaneous, transdermal, or topical administration, although carriers for oral administration are most preferred.

Pharmaceutically acceptable carriers for oral administration include capsules, tablets, pills, powders, troches and granules. In such solid dosage forms, the carrier can comprise at least one inert diluent such as sucrose, lactose or starch. Such carriers can also comprise, as is normal practice, additional substances other than diluents, e.g., lubricating agents such as magnesium stearate. In the case of capsules, tablets, troches and pills, the carriers may also comprise buffering agents. Carriers such as tablets, pills and granules can be prepared with enteric coatings on the surfaces of the tablets, pills or granules. Alternatively, the enterically coated compound can be pressed into a tablet, pill, or granule, and the tablet, pill or granules for administration to the patient. Preferred enteric coatings include those that dissolve or disintegrate at colonic pH such as shellac or Eudraget S.

Pharmaceutically acceptable carriers include liquid dosage forms for oral administration, e.g., pharmaceutically acceptable emulsions, solutions, suspensions, syrups and elixirs containing inert diluents commonly used in the art, such as water. Besides such inert diluents, compositions can also include adjuvants such as wetting agents, emulsifying and suspending agents, and sweetening, flavoring and perfuming agents.

Pharmaceutically acceptable carriers for topical administration include DMSO, alcohol or propylene glycol that can be employed with patches or other liquid-retaining material to hold the medicament in place on the skin so that the medicament will not dry out.

Pharmaceutically acceptable carriers for intravenous administration include solutions containing pharmaceutically acceptable salts or sugars. Pharmaceutically acceptable carriers for intramuscular or subcutaneous injection include pharmaceutically acceptable salts, oils or sugars.

When used in its acid form, a compound of Formula I useful in the practice of this invention can be employed in the form of a pharmaceutically acceptable salt of the acid. For example, sodium or potassium salts can be obtained by neutralizing with an equivalent base (alkali) metal hydroxide, mesylate or tosylate. When the active chemical is a base, it can be used as an acceptable formulation by neutralizing it with a suitable acid such as hydrochloric acid. Carriers such as solvents, water, buffers, alkanols, cyclodextrans and aralkanols can be used. Other auxiliary, non-toxic agents may be included, for example, polyethylene glycols, antimicrobial agents and wetting agents.

The pharmaceutically acceptable carrier and compounds of this invention are formulated into unit dosage forms for administration to a patient. The dosage levels of active ingredient (*i.e.,* compounds of this invention) in the unit dosage may be varied so as to obtain an amount of active ingredient effective to achieve activity in accordance with the desired method of administration (*i.e.,* oral or intravenous). The selected dosage level therefore depends upon the nature of the active compound administered, the route of administration, the desired duration of treatment, and other factors. If desired, the unit dosage may be such that the daily requirement for active compound is in one dose, or divided among multiple doses for administration, *e.g.*, two to four times per day.

The pharmaceutical compositions of this invention are preferably packaged in a container (*e.g.*, a box or bottle, or both) with suitable printed material (*e.g*., a package insert) containing indications, directions for use.

Examples 1-3 illustrate compounds useful in the practice of the claimed invention. Example 4 describes the clinical results with a 16 year-old patient with acne vulgaris who was treated with exisulind. Examples 5 and 6 illustrate cGMP PDE inhibitory activity of various compounds of the present invention.

### EXAMPLE 1

### α-(1-p-Methylsulfonylbenzylidene)-2-Methyl-5-Fluoro-3-Indenyl-Acetic Acid

### (A) p -fluoro-α-methylcinnamic acid.

p-fluorobenzaldehyde (200 g, 1.61 mole), propionic anhydride (3.5 g, 2.42 mole) and sodium propionate (155 g, 1.61 mole) are mixed in a 1 liter, three-necked flask flushed with nitrogen. The mixture is heated gradually in an oil-bath to 140°C. After 20 hours, the flask is cooled to 100°C and poured into 8 liters of water. The precipitate is dissolved by adding potassium hydroxide (302 g) in 2 liters of water. The aqueous solution is extracted with ether, and the ether extracts are washed with potassium hydroxide solution. The combined aqueous layers are filtered, acidified with concentrated HCl, and filtered; and the collected solid washed with water, thereby producing p-fluoro-α-methylcinnamic acid which is used as obtained.

### (B) p-fluoro-α-methylhydrocinnamic acid.

To p-fluoro-α-methylcinnamic acid (177.9 g, 0.987 mole) in 3.6 liters ethanol is added 11.0 g of 5% Pd/C and the mixture is reduced at room temperature under a hydrogen pressure of 40 p.s.i.; uptake is 31-32 lbs. (97% of theoretical). After the catalyst is filtered, the filtrate is concentrated *in vacuo* to give the product p-fluoro-α-methylhydrocinnamic acid that is used without weighing in next step.

### (C) 6-fluoro-2-methylindanone.

To polyphosphoric acid (932 g) at 70°C on the steam bath is added p-fluoro-α-methylhydrocinnamic acid (93.2 g, 0.5 mole) slowly with stirring. The temperature is gradually raised to 95°C, and the mixture is kept at that temperature for 1 hour. The mixture is allowed to cool and added to 2 liters of water. The aqueous layer is extracted with ether, the ether solution washed twice with saturated sodium chloride solution, 5% Na₂CO₃ solution, water, and then dried. The ether filtrate is concentrated with 200 g silica-gel, and added to a five pound silica-gel column packed with 5% ether-petroleum ether. The column is eluted with 5-10% ether-petroleum ether and followed by TLC to give 6-fluoro-2-methylindanone.

### (D) 5-fluoro-2-methylindenyl-3-acetic acid.

A mixture of 6-fluoro-2-methylindanone (18.4 g, 0.112 g. mole), cyanoacetic acid (10.5 g, 0.123 mole), acetic acid (6.6 g), and ammonium acetate (1.7 g) in dry toluene (15.5 ml) is refluxed with stirring for 21 hours, as the liberated water is collected in a Dean Stark trap. The toluene is concentrated, and the residue dissolved in 60 ml of hot ethanol and 14 ml of 2.2N aqueous potassium hydroxide solution. 22 g of 8.5% KOH in 150 ml of water is added, and the mixture refluxed for 13 hours under nitrogen. The ethanol is removed under vacuum, water (500 ml) is added, and the aqueous solution washed well with ether, and then boiled with charcoal. The aqueous filtrate is acidified to pH 2 with 50% hydrochloric acid, cooled, and the precipitate collected. In this way dried 5-fluoro-2-methylindenyl-3-acetic acid (M.P. 164 -166°C) is obtained.

### (E) 5-fluoro-2-methyl-1-(p-methylthiobenzylidene)-3-indenyl acetic acid.

5-fluoro-2-methyl-3-indenyl acetic acid (15 g, 0.072 mole) p-methylthiobenzaldehyde (14.0 g, 0.091 mole) and sodium methoxide (13.0 g, 0.24 mole) are heated in methanol (200 ml) at 60°C under nitrogen with stirring for 6 hours. After cooling, the reaction mixture is poured into ice-water (750 ml), acidified with 2.5N hydrochloric acid, and the collected solid triturated with a little ether to produce 5-fluoro-2-methyl-1-(p-methylthiobenzylidene)-3-indenyl acetic acid (M.P. 187-188.2°C). U.V. in methanol λmax. 348 (E % 500), 258 (557), 258 (495), 353 (513), 262.5 (577), 242.5 (511).

### (F) 5-fluoro-2-methyl-1-(p-methylsulfinylbenzylidene)-3-indenyl acetic acid.

To a solution of 5-fluoro-2-methyl-1-(p-methylthiobenzylidene)-3-indenyl acetic acid (3.4 g, 0.01 mole) in a mixture of methanol (250 ml) and acetone (100 ml) is added a solution of sodium periodate (3.8 g, 0.018 mole) in water (50 ml) with stirring. Water (450 ml) is added after 18 hours, and the organic solvents removed under vacuum below 30°C. The precipitated product is filtered, dried and recrystallized from ethyl acetate to give 5-fluoro-2-methyl-1-(rhomethylsulfinylbenzylidene)-3-indenyl acetic acid. Upon repeated recrystallization upon ethylacetate there is obtained cis-5-fluoro-2-methyl-1-(p-methylsulfinylbenzylidene)-3-indenyl acetic acid, M.P. 184-186°C. U.V. in methanol; λmax 328 (E % 377), 286 (432), 257.5 shldr. (413), 227 (548). Further runs reveal the existence of a second polymorph of cis-5-fluoro-2-methyl-1-(p-methylsulfinylbenzylidene)-3-indenyl acetic acid, M.P. 179-181°C.

### (G) 5-fluoro-2-methyl-1-(p-methylsulfonylbenzylidene)-3-indenyl acetic acid

5-fluoro-2-methyl-1-(p-methylsulfonylbenzylidene)-3-indenyl acetic acid is prepared by adding sodium methoxide (4.4M in MeOH, 68.5 ml, 0.3 mol) dropwise to a stirred, cooled mixture of 5-fluoro-2-methyl-1-(p-methylsulfinylbenzylidene)-3-indenyl acetic acid (100 g, 0.281 mol) in methanol (250 ml) and acetonitrile (500 ml). Sodium bicarbonate (0.56 mol) and hydrogen peroxide (30% in water, 0.56 mol) are added and allowed to react for 18 hours at -10°C. Excess sodium bicarbonate is filtered off, and cooled filtrate (0°C) neutralized dropwise to pH 7 with 1M hydrochloric acid (350 ml). The resulting product is then filtered and washed with methanol. A thin layer chromatography system to check for purity utilizes chloroform:methyl isobutyl ketone (8:2); the R_{f} value is 0.21. A tetrahydrofuran/diisopropyl ether combination can be used for product recrystallization. Reaction yield is 89%. (R₁ = 5-fluoro; R₂ = CH₃ ; R₃ = hydrogen; R₄ = COOH; R₅ = CH₃ SO₂; n = 1).
Formula: C₂₀H₁₇FO₄S
Molecular Mass: 372.41 g/mol
Melting point: 204-206°C
¹H-NNM [ppm] (DMSO-d₆): 2.16 (s,3,-CH₃); 3.30 (s,3,-SO₂-CH₃); 3.59 (s,2,-CH₂-C=O); 6.70-7.17 (m,3,ar.); 7.38 (s,1,=CH-); 7.78-8.04 (AB,4,-Ph-SO₂-).
HPLC (C-18 Column, 50% acetic acid (2%) / 50% acetonitrile, 1.5 ml/min): 3.21 min
IR [cm⁻¹] (KBr): 1710 C=O; 1310 S=O; 1180 C-F; 1140 S=O.

α-[1-(p-methylsulfonylbenzylidene)-2-methyl-5-fluoro-3-indenyl]-propionic acid is prepared by the similar procedures known in the art.

### EXAMPLE 2

### α-(1-p-Methylsulfonylbenzylidene)-2-Methyl-5-Fluoro-3-Indenyl -Acetic Acid Methyl Ester

5-fluoro-2-methyl-1-(p-methylsulfonylbenzylidene)-3-indenyl acetic acid is prepared by the procedure of Example 1, and converted to the methyl ester derivative by the following procedure. Sodium methoxide (4.4M in methanol, 1.36 ml, 0.006 mol) is added to a stirred cooled solution (0°C) of 5-fluoro-2-methyl-1-(p-methylsulfonylbenzylidene)-3-indenyl acetic acid (1.04 g, 0.0028 mol) in methanol (5 ml) and acetonitrile (10 ml). After 30 minutes, the reaction mixture is dropped into concentrated hydrochloric acid (50 ml) and extracted with methylene chloride (3 x 25 ml). The organic layer is extracted with saturated sodium bicarbonate (3 x 25 ml), dried with sodium sulfate, and concentrated *in vacuo.* The resulting oil is crystallized from tetrahydrofuran/hexane to yield 0.2 g of the desired compound. The melting point is 165 -166°C (R₁ = 5-fluoro; R₂ = CH₃; R₃ = hydrogen; R₄ = COO CH₃; R₅ = CH₃ SO₂; n =1 ).

Other methyl esters of compounds of Formula I useful in this invention can be prepared in a similar fashion.

### EXAMPLE 3

### (Z)-5-Fluoro-2-Methyl-1-(4-Methylsulfonylbenzylidene)-1H-3-Indenyl-(2-Hydroxy) Ethane

### (A) methyl-5-fluoro-2-methyl-1H-3-indenylacetate

Nitrosomethylurea (99.5 mmol) is added in portions to a cold (0°C) mixture of aqueous 50% KOH (50 ml) and diethylether (150 ml) at 0°C. The yellow ether solution of diazomethane (note: explosive) is separated, is washed with water, and is added in portions to a solution of 5-fluoro-2-methylindene-3-acetic acid (90 mmol) in dichloromethane (200 ml). When the evolution of N₂ ceases, the reaction is complete. After evaporation of the solvents, the residue is recrystallized from hexane to give methyl-5-fluoro-2-methyl-3-indenylacetate (yield 93%; M.P. 53°C).

### (B) 5-Fluoro-2-methyl-1H-3-indenyl-(2-hydroxy) ethane

To a solution of methyl-5-fluoro-2-methyl-3-indenyl-acetate (24 g) in dry THF (300 ml) lithiumaluminum hydride (6.9 g) is added. The mixture is stirred at room temperature for 1.5 hours. Excess LiAlH₄ is destroyed with saturated aqueous NaHSO₄ solution. The organic phase is concentrated *in vacuo,* and the crude product is purified via silica gel column chromatography elution with methylene chloride. The residue is recrystallized from hexane to give 5-fluoro-2-methyl-1H-3-indenyl-(2-hydroxy) ethane (yield 63%; M.P. 65°-66.5°C).

### (C) (Z)-5-fluoro-2-methyl-1-(4-methylsulfonylbenzylidene)-1H-3-indenyl-(2-hydroxy) ethane

5-fluoro-2-methyl-1H-3-indenyl-(2-hydroxy) ethane (15 g, 0.072 mol), p-methylsulfonylbenzaldehyde (14.0 g, 0.091 mol) and sodium methoxide (13.0 g, 0.24 mol) are heated in methanol (200 ml) at 60°C under nitrogen with stirring for 6 hours. The reaction mixture is poured onto ice-water (750 g), and is acidified with 2.5N hydrochloric acid. The collected solid is triturated with a little ether to produce (Z)-5-fluoro-2-methyl-1-(p-methylsulfonylbenzylidene)-1H-3-indenyl-(2-hydroxy) ethane. Recrystallization of the crude reaction product results in the separation of the mixture of geometrical isomers (Z/E) and gives the title compound (R₁ = 5-fluoro, R₂ = CH₃, R₃ = H, R₄ = CH₂OH, , n = 1, R₅ =CH₃ SO₂).
Formula: C₂₀H₁₉FO₃S
Molecular Mass: 358.43 g/mol
Melting point: 118°C
¹H-NMR [ppm] (DMSO-d₆): 2.14 (s,3,-CH₃); 2.71 (t, 2,-CH₂-); 3.29 (s,3,-SO₂-CH₃); 3.55 (m,3,-CH₂-O); 4.70 (m,1,-OH); 6.68-7.14 (m,3,ar.);7.30 (s,1,=CH); 7.76-8.03 (AB,4,-Ph-SO₂-);
IR [cm⁻¹] (KBr): 3440 OH; 1300 S=O; 1170 C-F; 1140 S=O

### EXAMPLE 4

### Clinical Results With A Patient With Acne Vulgaris

A 16 year-old male patient with acne vulgaris was given a pharmaceutical composition containing 125 mg of a cGMP-specific PDE inhibitor, exisulind (the compound of Example 1), twice daily for 8 weeks. At the end of that time, the patient's acne was observed to decrease noticeably in intensity. Since the patient was tolerating that daily dose quite well, his dose of exisulind was increased to 150 mg twice daily, for an additional 8 weeks. By the end this second 8-week period, the patient's acne had disappeared completely, and the patient reported no adverse side effects, clinically significant or not. The patient continued on drug for six months without any significant manifestations of acne during that time.

The pharmaceutical formulation for the 150 mg-containing pharmaceutical composition taken by this patient contained exisulind (150 mg), lactose monohydrate (207 mg), croscarmellose sodium (15 mg), colloidal silicon dioxide (1 mg), magnesium stearate (6.3 mg), and sodium lauryl sulfate (0.7 mg) encapsulated in a # 1 gelatin capsule. The exisulind was obtained from Zambon Group, S.p.A., of Milan, Italy. The pharmaceutical formulation for the 125 mg containing composition was formulated with less exisulind, and more excipients to fill the capsule.

Larger adult dosage forms simply contain more active ingredient, and less excipient. Pediatric or adult doses also may be formulated in tablet form of suitable dosages.

### Example 5

### cGMP-Specific PDE Inhibition Activity

cGMP-specific phosphodiesterase activity can be determined using methods known in the art, such as a method using radioactive ³H cyclic GMP (cyclic 3',5'-guanosine monophosphate) as the substrate for PDE5 enzyme (Thompson, W.J., Teraski, W.L., Epstein, P.M., Strada, S.J., *Advances in Cyclic Nucleotide Research,* 10:69-92, 1979. Using such techniques, the values of cGMP-specific inhibition set forth in Table 1 were determined for the compounds above:

**TABLE 1**

| **Compound** | **PDE5 % Inhibition at 10 µM** |
|---|---|
| Example 1 | 39 |
| Example 2 | 50 |
| Example 3 | 80 |

### Example 6

### cGMP-Stimulated PDE Inhibition Activity

cGMP phosphodiesterase activity can be determined using the methods referenced in Example 5, above. Using such techniques, the IC₅₀ value for the inhibition of PDE2 by the compound of Example 1, exisulind, was determined as shown in Table 2.

**TABLE 2**

| **Compound** | **IC**_{**50**} **for PDE2** |
|---|---|
| Example 1 | 300µm |

## Claims

1. Use of a compound of the formula: 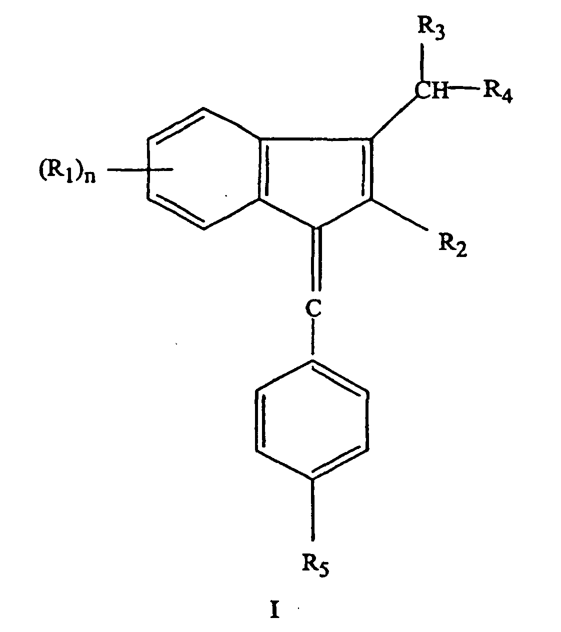 wherein R₁ is independently selected in each instance from the group consisting of hydrogen, halogen, alkoxy having 1 to 8 carbons, hydroxy, alkyl having 1 to 8 carbons, alkyl mercapto having 1 to 8 carbons, alkylsulfonyl having 1 to 8 carbons, alkylamino having 1 to 8 carbons, di-alkyl amino having 1 to 8 carbons, amino, nitro, nitrile, alkyl carboxylate having 1 to 8 carbons, -CO₂H, and sulfonamido;
R₂ is selected from the group consisting of hydrogen and alkyl having 1 to 8 carbons;
R₃ is selected from the group consisting of hydrogen, alkyl having 1 to 8 carbons, hydroxy, and amino;
R₄ is selected from the group consisting of -COM and CH₂OH wherein M is selected from the group consisting of hydroxy, substituted alkoxy having 1 to 8 carbons, amino, alkylamino, dialkylamino, N-morpholino, hydroxyalkylamino, polyhydroxyamino, dialkylaminoalkylamino, aminoalkylamino, and the group OMe, wherein Me is a cation;
R₅ is an alkyl sulfonyl; and n is an integer from 0 to four;
or a pharmaceutically acceptable salt thereof, in the manufacture of a medicament for the treatment of acne.

2. Use according to claim 1 wherein R₁ is halogen and n is 1.

3. Use according to claim 2 wherein R₁ is 5-fluoro.

4. Use according to claim 2 wherein R₂ is alkyl having 1 to 8 carbons.

5. Use according to claim 4 wherein R₂ is methyl.

6. Use according to claim 4 wherein M is hydroxy; and R₃ is selected from the group consisting of hydrogen or alkyl having 1 to 8 carbons.

7. Use according to claim 6 wherein R₃ is hydrogen.

8. Use according to claim 1 wherein the compound is (Z)-5-nuoro-2-methyl-1-(p-methylsulfonylbenzylidene)-3-indenyl acetic acid or a pharmaceutically acceptable salt thereof.

9. Use of a physiological effective amount of a compound selected from the group:
α-(1-p-methylsulfonylbenzylidene)-2-methyl-5-fluoro-3-indenyl -acetic acid,
α-[1-(p-methylsulfonylbenzylidene)-2-methyl-5-fluoro-3-indenyl]-propionic acid,
α-(1-p-methylsulfonylbenzylidene)-2-methyl-5-fluoro-3-indeny-1-acetic acid methyl ester, and
(Z)-5-fluoro-2-methyl-1-(4-methylsulfonylbenzylidene)-1H-3-indenyl- (2-hydroxy) ethane,
or a pharmaceutically acceptable salt thereof in the manufacture of a medicament for treating a patient with acne.

10. Use according to claim 1 wherein the compound is (Z)-5-fluoro-2-methyl-(4-pyridylidene)-3-(N-benzyl) indenylacetamide hydrochloride.

## Patentansprüche

1. Verwendung einer Verbindung der Formel: 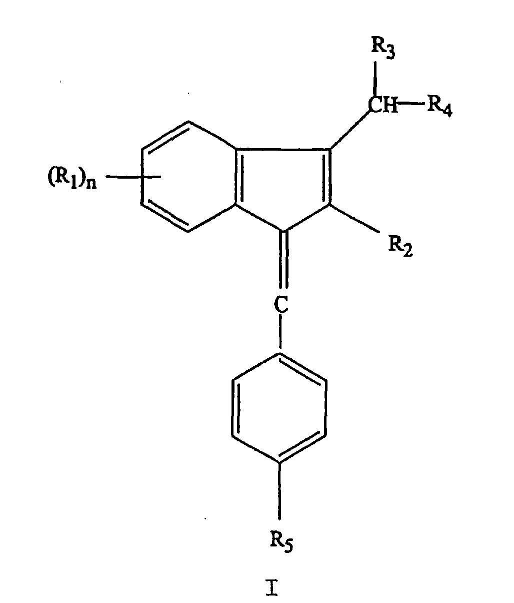 wobei R₁ jeweils unabhängig ausgewählt ist aus der Gruppe bestehend aus Wasserstoff, Halogen, Alkoxy mit 1 bis 8 Kohlenstoffen, Hydroxy, Alkyl mit 1 bis 8 Kohlenstoffen, Alkylmerkapto mit 1 bis 8 Kohlenstoffen, Alkylsulfonyl mit 1 bis 8 Kohlenstoffen, Alkylamino mit 1 bis 8 Kohlenstoffen, Dialkylamino mit 1 bis 8 Kohlenstoffen, Amino, Nitro, Nitril, Alkylcarboxylat mit 1 bis 8 Kohlenstoffen, -CO₂H und Sulfonamido;
R₂ ausgewählt ist aus der Gruppe bestehend aus Wasserstoff und Alkyl mit 1 bis 8 Kohlenstoffen;
R₃ ausgewählt ist aus der Gruppe bestehend aus Wasserstoff, Alkyl mit 1 bis 8 Kohlenstoffen, Hydroxy und Amino;
R₄ ausgewählt ist aus der Gruppe bestehend aus -COM und CH₂OH, wobei M ausgewählt ist aus der Gruppe bestehend aus Hydroxy, substituiertem Alkoxy mit 1 bis 8 Kohlenstoffen, Amino, Alkylamino, Dialkylamino, N-Morpholino, Hydroxyalkylamino, Polyhydroxyamino, Dialkylaminoalkylamino, Aminoalkylamino und der Gruppe OMe, wobei Me ein Kation ist;
R₅ ein Alkylsulfonyl ist und n eine Ganzzahl von 0 bis vier ist;
oder eines pharmazeutisch akzeptablen Salzes davon zur Herstellung eines Medikaments zur Behandlung von Akne.

2. Verwendung gemäß Anspruch 1, wobei R₁ Halogen ist und n 1 ist.

3. Verwendung gemäß Anspruch 2, wobei R₁ 5-Fluor ist.

4. Verwendung gemäß Anspruch 2, wobei R₂ Alkyl mit 1 bis 8 Kohlenstoffen ist.

5. Verwendung gemäß Anspruch 4, wobei R₂ Methyl ist.

6. Verwendung gemäß Anspruch 4, wobei M Hydroxy ist und R₃ ausgewählt ist aus der Gruppe bestehend aus Wasserstoff oder Alkyl mit 1 bis 8 Kohlenstoffen.

7. Verwendung gemäß Anspruch 6, wobei R₃ Wasserstoff ist.

8. Verwendung gemäß Anspruch 1, wobei die Verbindung (Z)-5-Fluor-2-methyl-1-(p-methylsulfonylbenzyliden)-3-indenylessigsäure oder ein pharmazeutisch akzeptables Salz davon ist.

9. Verwendung einer physiologisch wirksamen Menge einer Verbindung ausgewählt aus der Gruppe:
α-(1-p-Methylsulfonylbenzyliden)-2-methyl-5-fluor-3-indenylessigsäure,
α-[1-(p-Methylsulfonylbenzyliden)-2-methyl-5-fluor-3-indenyl]propionsäure,
α-(1-p-Methylsulfonylbenzyliden)-2-methyl-5-fluor-3-indenylessigsäure-methylester und
(Z)-5-Fluor-2-methyl-1-(4-methylsulfonylbenzyliden)-1 H-3-indenyl-(2-hydroxy)ethan,
oder eines pharmazeutisch akzeptablen Salzes davon zur Herstellung eines Medikaments zur Behandlung eines Patienten mit Akne.

10. Verwendung gemäß Anspruch 1, wobei die Verbindung (Z)-5-Fluor-2-methyl-(4-pyridyliden)-3-(N-benzyl)indenylacetamid-Hydrochlorid ist.

## Revendications

1. Utilisation d'un composé de formule : 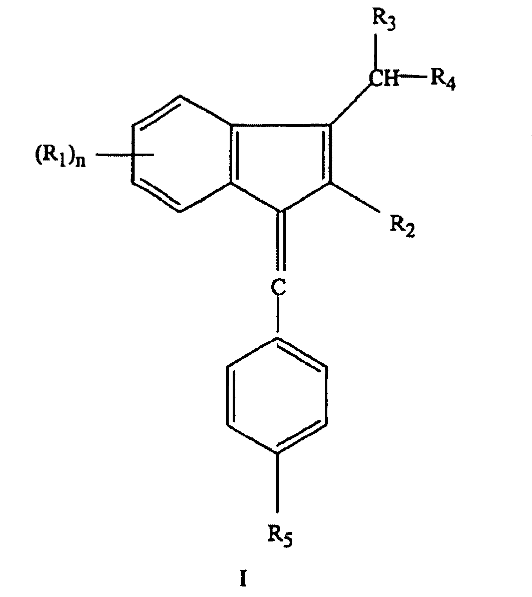 dans laquelle R₁ est choisi indépendamment dans chaque cas dans le groupe consistant en un atome d'hydrogène, un atome d'halogène, des groupes alkoxy ayant 1 à 8 atomes de carbone, hydroxy, alkyle ayant 1 à 8 atomes de carbone, alkylmercapto ayant 1 à 8 atomes de carbone, alkylsulfonyle ayant 1 à 8 atomes de carbone, alkylamino ayant 1 à 8 atomes de carbone, dialkylamino ayant 1 à 8 atomes de carbone, amino, nitro, nitrile, alkylcarboxylate ayant 1 à 8 atomes de carbone, -CO₂H et sulfonamido ;
R₂ est choisi dans le groupe consistant en un atome d'hydrogène et un groupe alkyle ayant 1 à 8 atomes de carbone ;
R₃ est choisi dans le groupe consistant en un atome d'hydrogène, des groupes alkyle ayant 1 à 8 atomes de carbone, hydroxy et amino ;
R₄ est choisi dans le groupe consistant en des groupes -COM et CH₂OH dans lesquels M est choisi dans le groupe consistant en des groupes hydroxy, alkoxy substitué ayant 1 à 8 atomes de carbone, amino, alkylamino, dialkylamino, N-morpholino, hydroxyalkylamino, polyhydroxyamino, dialkylaminoalkylamino, aminoalkylamino et le groupe OMe, dans lequel Me représente un cation ;
R₅ représente un groupe alkylsulfonyle ; et n représente un nombre entier de 0 à 4 ;
ou d'un de ses sels pharmaceutiquement acceptables, dans la production d'un médicament destiné au traitement de l'acné.

2. Utilisation suivant la revendication 1, dans laquelle R₁ représente un atome d'halogène et n est égal à 1.

3. Utilisation suivant la revendication 2, dans laquelle R₁ représente un groupe 5-fluoro.

4. Utilisation suivant la revendication 2, dans laquelle R₂ représente un groupe alkyle ayant 1 à 8 atomes de carbone.

5. Utilisation suivant la revendication 4, dans laquelle R₂ représente un groupe méthyle.

6. Utilisation suivant la revendication 4, dans laquelle M représente un groupe hydroxy et R₃ est choisi dans le groupe consistant en un atome d'hydrogène et un groupe alkyle ayant 1 à 8 atomes de carbone.

7. Utilisation suivant la revendication 6, dans laquelle R₃ représente un atome d'hydrogène.

8. Utilisation suivant la revendication 1, dans laquelle le composé est l'acide (Z)-5-fluoro-2-méthyl-1-(p-méthylsulfonylbenzylidène)-3-indénylacétique ou un de ses sels pharmaceutiquement acceptables.

9. Utilisation d'une quantité physiologiquement efficace d'un composé choisi dans le groupe :
acide α-(1-p-méthylsulfonylbenzylidène)-2-méthyl-5-fluoro-3-indényl-acétique,
acide α-[1-(p-méthylsulfonylbenzylidène)-2-méthyl-5-fluoro-3-indényl]-propionique,
ester méthylique d'acide α-(1-p-méthylsulfonylbenzylidène)-2-méthyl-5-fluoro-3-indényl-1-acétique, et
(Z)-5-fluoro-2-méthyl-1-(4-méthylsulfonylben,zylidène)-1H-3-indényl-(2-hydroxy)éthane,
ou d'un de ses sels pharmaceutiquement acceptables dans la production d'un médicament destiné au traitement d'un patient présentant de l'acné.

10. Utilisation suivant la revendication 1, dans laquelle le composé est le chlorhydrate de (Z)-5-fluoro-2-méthyl-(4-pyridylidène)-3-(N-benzyl)indénylacétamide.
